# EUROPEAN PATENT SPECIFICATION

(11) **EP 3 294 260 B1**
(45) Date of publication and mention of the grant of the patent: **09.10.2019**
(21) Application number: 16718353.2
(22) Date of filing: 25.04.2016
(51) Int. Cl.: A61P 1/02, A61K 8/27, A61K 8/25, A61K 8/28, A61K 8/29, A61Q 11/00, A61K 8/02

(54) **ORAL CARE COMPOSITION**
MUNDPFLEGEZUSAMMENSETZUNG
COMPOSITION DE PROTECTION ORALE

(30) Priority: 13.05.2015 WO PCT/CN2015/078832; 23.06.2015 EP 15173258
(43) Date of publication of application: 21.03.2018
(73) Proprietor: Unilever N.V., 3013 AL Rotterdam (NL); Unilever PLC, London, Greater London EC4Y 0DY (GB)
(72) Inventor: LI, Xiaoke, Shanghai 200335 (CN); LIU, Weining, Shanghai 200335 (CN); WANG, Jinfang, Shanghai 200335 (CN); ZHANG, Meili, Shanghai 200335 (CN)
(74) Representative: Tansley, Sally Elizabeth
(86) International application number: PCT/EP2016/059129
(87) International publication number: WO 2016/180621

(56) References cited:
- EP-A1- 0 269 966
- EP-A1- 2 572 698
- WO-A1-02/30381
- WO-A1-2015/036277
- WO-A1-2015/036285
- WO-A2-2012/031786
- FR-A1- 2 843 748

## Description

### Technical Field of the Invention

The present invention relates to oral care compositions such as tooth pastes, gums, mouthwashes and the like. In particular the present invention relates to an oral care composition that results in whitening and/or remineralization of teeth by delivering calcium, silicate source and a particulate tooth whitening agent to the teeth of an individual when, for example, brushing teeth. The invention also relates to the use of such compositions for whitening and/or remineralizing of teeth of an individual.

### Background of the Invention

The enamel layer of the tooth is naturally an opaque white or slightly off-white colour. However, this enamel layer can become stained or discoloured. The enamel layer of the tooth is composed of hydroxyapatite mineral crystals that create a somewhat porous surface. It is believed that this porous nature of the enamel layer allows staining agents and discolouring substances to permeate the enamel and discolour the tooth.

Many products we consume have a negative impact on our teeth and mouth. Acidic drinks and sweets, for example, can result in tooth erosion by attacking enamel which is the outer coating that protects the teeth. Moreover, tobacco based products as well as beverages like coffee and tea can stain or reduce the whiteness of one's teeth. These staining and discolouring substances are often able to permeate the enamel layer. This problem occurs gradually over many years, but imparts a noticeable discoloration of the enamel of one's teeth.

Consumers have always had a strong desire for white teeth and many individuals are dissatisfied with their current teeth colour. This desire for whiter teeth has given rise to a growing trend in the increased use of tooth whitening products.

A variety of products are currently used for teeth whitening. Such products often comprise peroxides, abrasives or both in order to clean and whiten teeth. These types of products are often not desired since they do not contribute to the remineralization of teeth and can cause damage to teeth and gums if overused. Products comprising calcium source have been developed in an attempt to enhance the characteristics of teeth. Such products, however, do not positively adhere to teeth and thereby may only contact teeth for a brief period of time prior to being rinsed out of the mouth in wash water.

The present inventors have now recognized a need to develop an oral care composition which has particulate tooth whitening agents that are suitable to adhere to the tooth surface to provide whitening benefits, particularly, the oral care composition is suitable to deliver calcium and silicate sources to teeth and form calcium silicate hydrate *in situ* that unexpectedly aids the deposition of particulate whitening agents. Additionally, it has been found unexpectedly that such *in situ* formed calcium silicate hydrate behaves much more active than pre-formed calcium silicate hydrate for tooth remineralization and/or whitening. Besides, by using *in situ* formed calcium silicate hydrate instead of pre-formed calcium silicate hydrate, it helps to simplify the manufacturing process and reduce the cost.

### Additional Information

WO 2008/068149 A (Unilever) discloses an oral care product comprising a first composition comprising an insoluble calcium salt that is not a calcium phosphate salt, a second independent composition comprising a source of phosphate ions, and a means for delivering each of the compositions to the surface of the teeth. The preferred insoluble calcium salt is calcium silicate.

WO 2012/031785 A (Unilever) discloses composite particle actives suitable for use in oral care compositions. The composite particle actives have a core and a coating whereby the coating interacts with phosphate ions to produce calcium and phosphate reaction products that are suitable to adhere to tooth enamel and/or dentin in order to improve characteristics of teeth.

WO 2012/031786 A (Unilever) discloses oral care compositions with composite particle actives described in WO 2012/031785 A (Unilever).

None of the references above describes an oral care composition comprising a calcium source and a water soluble silicate source, wherein the calcium source reacts with the silicate source to *in situ* produce calcium silicate hydrate that aids the deposition of tooth whitening agents.

### Tests and Definitions

### Dentifrice

"Dentifrice" for the purposes of the present invention means a paste, powder, liquid, gum or other preparation for cleaning the teeth or other surfaces in the oral cavity.

### Tooth Paste

"Tooth paste" for the purpose of the present invention means a paste or gel dentifrice for use with a toothbrush. Especially preferred are tooth pastes suitable for cleaning teeth by brushing for about two minutes.

### Mouth Wash

"Mouth wash" for the purpose of the present invention means liquid dentifrice for use in rinsing the mouth. Especially preferred are mouth washes suitable for rinsing the mouth by swishing and/or gargling for about half a minute before expectorating.

### Particle Size

"Particle size", as used herein, refers to particle diameter unless otherwise stated. Diameter is meant to mean the largest measurable distance or a leading dimension on a particle in the event a well-defined sphere is not generated. Particle size can be measured, for example, by dynamic light scattering (DLS).

### Composite Particle

"Composite particle", as used herein, refers to a particle comprising a first component core and a second component coating wherein the core and coating are composed of different materials.

### Refractive Index

Refractive index is quoted at a temperature of 25°C and a wavelength of 589 nm.

### Solubility

"Soluble" and "insoluble", as used herein, refers to the solubility of a source (e.g., like calcium salts) in water at 25°C and atmospheric pressure. "Soluble" means a source that dissolves in water to give a solution with a concentration of at least 0.1 moles per litre. "Insoluble" means a source that dissolves in water to give a solution with a concentration of less than 0.001 moles per litre. "Slightly soluble", therefore, is defined to mean a source that dissolves in water to give a solution with a concentration of higher than 0.001 moles per litre and less than 0.1 moles per litre.

### Substantially Free

"Substantially free of', as used herein, means less than 1.5%, and preferably less than 1.0%, and more preferably less than 0.75% and more preferably still less than 0.5%, and even more preferably less than 0.1% and most preferably from 0.0 to 0.01 % by weight, based on total weight of the oral care composition, including all ranges subsumed therein.

### Hydrous Composition

"Hydrous composition", as used herein, means the composition comprises water higher than 1.5%, preferably higher than 5%, more preferably higher than 10% and most preferably from 20 to 90% by weight of the composition.

### Viscosity

Viscosity of a tooth paste is the value taken at room temperature (25°C) with a Brookfield Viscometer, Spindle No.4 and at a speed of 5 rpm. Values are quoted in centipoises (cP=mPa.s) unless otherwise specified.

### In Situ

"*In situ*", as used herein, means during and/or after application of the oral care composition to the oral cavity.

### Remineralization

"Remineralization", as used herein, means *in situ* (i.e. in the oral cavity) generation of calcium phosphate on teeth (including layers on teeth from 10 nm to 20 microns, and preferably from 75 nm to 10 microns, and most preferably, from 150 nm to 5 microns thick including all ranges subsumed therein) to reduce the likelihood of tooth sensitivity, tooth decay, regenerate enamel and/or improve the appearance of teeth by whitening through the generation of such new calcium phosphate.

### Deposition Aid

Deposition aid, as used herein, means a material which aids deposition of particulate tooth whitening agent from the continuous phase of an oral care composition onto the tooth surface during use of the composition.

### Miscellaneous

Except in the examples, or where otherwise explicitly indicated, all numbers in this description indicating amounts of material or conditions of reaction, physical properties of materials and/or use may optionally be understood as modified by the word "about".

All amounts are by weight of the final oral care composition, unless otherwise specified.

It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

### Summary of the Invention

In a first aspect, the present invention is directed to an oral care composition comprising:
a) a calcium source;
b) a silicate source that dissolves in water to give a solution with a concentration of at least 0.1 moles per litre at 25°C and atmospheric pressure;
c) from 10% to 30% by weight of high refractive index particles; and
d) a physiologically acceptable carrier;
wherein the high refractive index particles have a refractive index ranging from 1.9 to 4.0,
wherein the calcium source is calcium hydroxide, calcium gluconate, calcium oxide, calcium lactate, calcium sulfate, calcium carboxymethyl cellulose, calcium alginate, calcium salts of citric acid, calcium chloride, calcium nitrate, calcium acetate or mixtures thereof; and
wherein the silicate source comprises sodium silicate, potassium silicate, tetraethyl orthosilicate, tetraethylsilicate or mixtures thereof.

In another aspect, the the present invention is directed to an oral care composition for use in a method of whitening and/or remineralizing the teeth of an individual comprising the step of applying the oral care composition of any embodiment of the first aspect to at least one surface of the teeth of the individual.

All other aspects of the present invention will more readily become apparent upon considering the detailed description and examples which follow.

### Detailed Description

Now it has been found that calcium source reacts with silicate source to *in situ* form calcium silicate hydrate that aids the deposition of particulate whitening agents such as high refractive index particles on tooth surfaces which delivers excellent tooth whitening benefits. It has also been found unexpectedly that such *in situ* formed calcium silicate hydrate behaves much more active than pre-formed calcium silicate hydrate for tooth whitening and/or remineralization. Besides, by using *in situ* formed calcium silicate hydrate instead of pre-formed calcium silicate hydrate, it helps to simplify the manufacturing process and reduce the cost.

The calcium source suitable for use in this invention is limited only to the extent that the same may be used in the mouth. The types of calcium source include calcium hydroxide, calcium gluconate, calcium oxide, calcium lactate, calcium sulfate, calcium carboxymethyl cellulose, calcium alginate, calcium salts of citric acid, calcium chloride, calcium nitrate, calcium acetate, mixtures thereof or the like. In a preferred embodiment, the calcium source is calcium hydroxide, calcium chloride, calcium nitrate, calcium sulfate, mixtures thereof or the like.

Typically, the oral care composition of the present invention comprises from 0.1 to 50% by weight of the calcium source, more preferably from 0.2 to 30%, most preferably from 1 to 20%, based on the total weight of the oral care composition and including all ranges subsumed therein.

The silicate source suitable for use in this invention is limited only to the extent that the same may be used in the mouth. In a preferred embodiment, the silicate source is water soluble. The silicate source suitable for use in this invention include sodium silicate, potassium silicate, tetraethyl orthosilicate, tetraethylsilicate, mixtures thereof or the like. In a preferred embodiment, the silicate source is sodium silicate.

Typically, the silicate source makes up from 0.5 to 50%, more preferably from 2 to 40%, and most preferably from 5 to 35% by weight of the oral care composition, based on total weight of the oral care composition and including all ranges subsumed therein.

The oral care composition preferably comprises the calcium source and silicate source in a molar ratio (Ca:Si) less than 1:1, more preferably less than 1:1.2, more preferably still from 1:1.5to 1:4 and most preferably from 1:1.7to 1:3.

The only limitation with respect to the high refractive index particles for use in this invention is that the same is suitable for use in the mouth.

In order to provide excellent whitening effect, the particles of the present invention are preferred to have a high refractive index of at least 1.9, more preferably at least 2.0, even more preferably at least 2.2, even more preferably still at least 2.4 and most preferably at least 2.5. The maximum refractive index of the high refractive index particles is not particularly limited but preferably up to 4.0.

Typically, the high refractive index particle comprises a material suitable to physically and immediately improve characteristics of teeth and especially whiten teeth. Particularly suitable materials are metal salts and preferred are salts where the metal is selected from zinc (Zn), titanium (Ti), zirconium (Zr) or a combination thereof. Preferably, the metal salts is (or at least comprises) a metal oxide such as titanium dioxide (TiO₂), zinc oxide (ZnO), zirconium dioxide (ZrO₂) or a combination thereof. In addition, the high refractive index particle can also comprise non-metal oxides such as silicon monoxide (SiO).

In a preferred embodiment, the high refractive index particle comprises metal oxides, non-metal oxides or a combination thereof in an amount of at least 50% by weight of the particle and more preferably at least 70%, more preferably still from 80 to 100% and most preferably from 85 to 95%. In an especially preferred embodiment, the high refractive index particle is at least 50% by weight titanium dioxide, and most preferably, from 60 to 100% by weight titanium dioxide, based on total weight of the high refractive index particle and including all ranges subsumed therein. In another especially preferred embodiment, the high refractive index particles are slightly soluble or insoluble in water, but most preferably, insoluble in water.

In a preferred embodiment, the high refractive index particles are composite particles. The refractive index of a composite particle comprising more than one material can be calculated based on the refractive indices and volume fractions of the constituents using effective medium theory, as is described for example in WO 2009/023353.

The composite particle comprises a first component core and a second component coating. Typically, the core of the composite particle comprises a material suitable to physically and immediately improve characteristics of teeth and especially whiten teeth. Particular suitable materials are metal salts and preferred are salts where the metal is selected from zinc (Zn), titanium (Ti), zirconium (Zr) or a combination thereof. Preferably, the metal salts is (or at least comprises) a metal oxide such as titanium dioxide (TiO₂), zinc oxide (ZnO), zirconium dioxide (ZrO₂) or a combination thereof. In addition, the core of the composite particle can also comprise non-metal oxides such as silicon monoxide (SiO).

The core of the composite particle typically makes up from 3 to 98%, and preferably from 6 to 65%, and most preferably from 10 to 55% by weight of the composite particle, based on total weight of the composite particle and including all ranges subsumed therein. In a preferred embodiment, the core comprises metal oxides, non-metal oxides or a combination thereof in an amount of at least 50% by weight of the core and more preferably at least 70%, more preferably still from 80 to 100% and most preferably from 85 to 95%. In an especially preferred embodiment, the core is at least 50% by weight titanium dioxide, and most preferably, from 60 to 100% by weight titanium dioxide, based on total weight of the first component core.

The second component coating comprises material suitable to adhere to tooth enamel, dentin or both. Typically the coating material comprises the element calcium, and optionally, other metals like potassium, sodium, aluminium, magnesium as well as mixtures thereof whereby such optional metals are provided as, for example, sulphates, lactates, oxides, carbonates or silicates. Optionally, the coating material may be aluminium oxide or silica. In a preferred embodiment, the coating material is suitable to provide a biological or chemical improvement to teeth which is long term (e.g., results in hydroxyapatite formation). Preferably, the coating employed comprises at least 50% by weight elemental calcium, and most preferably, at least 65% by weight elemental calcium based on total weight of metal in the coating. In an especially preferred embodiment, the metal in the coating is from 80 to 100% by weight of elemental calcium, based on total weight of metal in the second component coating and including all above-mentioned ranges subsumed therein. In another especially preferred embodiment, the core and the coating are slightly soluble or insoluble in water, but most preferably, insoluble in water.

In an especially desired embodiment, the second component coating can comprise, for example, calcium phosphate, calcium gluconate, calcium oxide, calcium lactate, calcium carbonate, calcium hydroxide, calcium sulphate, calcium carboxymethyl cellulose, calcium alginate, calcium slats of citric acid, calcium silicate, mixture thereof or the like. In another desired embodiment, the calcium source in the coating comprises calcium silicate.

In yet another preferred embodiment, the coating can comprise the element calcium which originates from insoluble calcium silicate, present as the composite material calcium oxide-silica (CaO-SiO₂) as described in international patent applications published as WO 2008/015117 and WO 2008/068248.

When a calcium silicate composite material is employed as coating, the ratio of calcium to silicon (Ca:Si) may be from 1:10 to 3:1. The Ca:Si ratio is preferably from 1:5 to 2:1, and more preferably, from 1:3 to 2:1, and most preferably, from about 1:2 to 2:1. The calcium silicate may comprise mono-calcium silicate, bi-calcium silicate, or tri-calcium silicate whereby ratios of calcium to silicon (Ca:Si) should be understood to be atom ratios.

Usually, at least 30% of the outer surface area of the first component core is coated with the second component coating, preferably at least 50% of the core is coated with the coating, most preferably, from 70 to 100% of the outer surface area of the first component core is coated with the second component coating.

In an especially preferred embodiment, the high refractive index particle is titanium dioxide coated with calcium silicate.

The high refractive index particles according to the present invention can be of different sizes and shapes. The particles may be of spherical, platelet or irregular shape form. The diameter of the high refractive index particles is often from 10 nm to less than 50 microns, and preferably, from 75 nm to less than 10 microns. In an especially preferred embodiment, the diameter of particles is from 100 nm to 5 microns, including all ranges subsumed therein. For composite particles, in a preferred embodiment, at least 40%, and preferably, at least 60%, and most preferably, from 75 to 99.5% of the diameter of the composite particle is the core, including all ranges subsumed therein.

Typically, the oral care composition of the present invention comprises from 2 to 30%, and preferably, from 5 to 25%, and most preferably, from 10 to 20% by weight of the high refractive index particles, based on the total weight of the oral care composition and including all ranges subsumed therein.

The high refractive index particles of the present invention may be capable of adhering to tooth surfaces in the presence of *in situ* formed calcium silicate hydrate without inclusion of a phosphate source in the composition itself. Without wishing to be bound by the theory the present inventors believe that this may be because the Si-OH groups may have an affinity for Ca ions in teeth, and the *in situ* formed calcium silicate hydrate reacts with phosphate ions in saliva that leads to hydroxyapatite formation. The remineralization of calcium silicate around the particles helps the deposition of high refractive index particles on tooth surface by enhancing their resistance to shear force.

Thus in one embodiment the composition may be substantially free of phosphate source.

For anhydrous compositions (i.e. compositions substantially free of water) or a dual phase hydrous compositions, it is preferred to compound a phosphate source in the composition.

The phosphate source that may be used in this invention is limited only to the extent that the same may be used in a composition suitable for use in the mouth. Illustrative examples of the types of phosphate source suitable for use in this invention include trisodium phosphate, monosodium dihydrogen phosphate, disodium hydrogen phosphate, sodium pyrophosphate, tetrasodium pyrophosphate, sodium hexametaphosphate, tripotassium phosphate, monopotassium dihydrogen phosphate, dipotassium hydrogen phosphate, mixtures thereof or the like. The phosphate source is preferably one which is water soluble.

When used, the phosphate source typically makes up from 0.5 to 22%, and preferably, from 2 to 18%, and most preferably, from 4 to 16% by weight of the oral care composition, based on total weight of the oral care composition and including all ranges subsumed therein. In a preferred embodiment, the phosphate source used is trisodium phosphate and monosodium dihydrogen phosphate at a trisodium phosphate to monosodium dihydrogen phosphate weight ratio of 1:4 to 4:1, preferably 1:3 to 3:1, and most preferably, from 1:2 to 2:1, including all ratios subsumed therein.

The composition of the present invention is an oral care composition and typically comprises physiologically acceptable carrier. The carrier preferably comprises at least surfactant, thickener, humectant or a combination thereof.

Preferably the oral care composition comprises a surfactant. Preferably the composition comprises at least 0.01 % surfactant by weight of the composition, more preferably at least 0.1% and most preferably from 0.5 to 7%. Suitable surfactants include anionic surfactants, such as the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers.

Other suitable surfactants include amphoteric surfactants, such as betaines or sulphobetaines. Mixtures of any of the above described materials may also be used. More preferably the surfactant comprises or is anionic surfactant. The preferred anionic surfactants are sodium lauryl sulphate and/or sodium dodecylbenzene sulfonate. Most preferably the surfactant is sodium lauryl sulphate.

Thickener may also be used in this invention and is limited only to the extent that the same may be added to a composition suitable for use in the mouth. Illustrative examples of the types of thickeners that may be used in this invention include, sodium carboxymethyl cellulose (SCMC), hydroxyl ethyl cellulose, methyl cellulose, ethyl cellulose, gum tragacanth, gum arabic, gum karaya, sodium alginate, carrageenan, guar, xanthan gum, Irish moss, starch, modified starch, silica based thickeners including silica aerogels, magnesium aluminum silicate (e.g., Veegum), Carbomers (cross-linked acrylates) and mixtures thereof.

Typically, xanthan gum and/or sodium carboxymethyl cellulose and/or a Carbomer is/are preferred. When a Carbomer is employed, those having a weight-average molecular weight of at least 700,000 are desired, and preferably, those having a molecular weight of at least 1,200,000, and most preferably, those having a molecular weight of at least about 2,500,000 are desired. Mixtures of Carbomers may also be used herein.

In an especially preferred embodiment, the Carbomer is Synthalen PNC, Synthalen KP or a mixture thereof. It has been described as a high molecular weight and cross-linked polyacrylic acid and identified via CAS number 9063-87-0. These types of materials are available commercially from suppliers like Sigma.

In another especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

In another especially preferred embodiment, the thickener is xanthan gum.

Thickener typically makes up from 0.01 to about 10%, more preferably from 0.1 to 9%, and most preferably, from 1.5 to 8% by weight of the oral care composition, based on total weight of the composition and including all ranges subsumed therein.

When the oral care composition of this invention is a toothpaste or gel, the same typically has a viscosity from about 30,000 to 180,000 centipoise, and preferably, from 60,000 to 170,000 centipoise, and most preferably, from 65,000 to 165,000 centipoise.

Suitable humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol, sorbitol or mixtures thereof are the preferred humectants.

The humectant may be present in the range of from 10 to 90% by weight of the oral care composition. More preferably, the carrier humectant makes up from 25 to 80%, and most preferably, from 45 to 70% by weight of the composition, based on total weight of the composition and including all ranges subsumed therein.

The oral care composition of the present invention may contain a variety of other ingredients which are common in the art to enhance physical properties and performance. These ingredients include antimicrobial agents, anti-inflammatory agents, anti-caries agents, plaque buffers, fluoride sources, vitamins, plant extracts, desensitizing agents, anti-calculus agents, biomolecules, flavors, proteinaceous materials, preservatives, opacifying agents, coloring agents, pH-adjusting agents, sweetening agents, particulate abrasive materials, polymeric compounds, buffers and salts to buffer the pH and ionic strength of the compositions, and mixtures thereof. Such ingredients typically and collectively make-up less than 20% by weight of the composition, and preferably, from 0.0 to 15% by weight, and most preferably, from 0.01 to 12% by weight of the composition, including all ranges subsumed therein.

The oral care composition of this invention is for use in a method of whitening and/or remineralizing of teeth of an individual comprising applying the composition to at least one surface of the teeth of the individual.

In a preferred embodiment, the oral care composition is a monophase anhydrous composition. In another preferred embodiment, the oral care composition is a dual phase composition, wherein the calcium source and the soluble silicate source are spatially separated. The calcium source and silicate source are applied to the teeth of the individual from spatially separated compositions simultaneously or sequentially, preferably simultaneously.

Typically the composition will be packaged. In tooth paste or gel form, the composition may be packaged in a conventional plastic laminate, metal tube or a single compartment dispenser. The same may be applied to dental surfaces by any physical means, such as a toothbrush, fingertip or by an applicator directly to the sensitive area. In liquid mouthwash form the composition may be packaged in a bottle, sachet or other convenient container.

The composition can be effective even when used in an individual's daily oral hygiene routine. For example, the composition may be brushed onto the teeth and/or be rinsed around the inside of the mouth of the individual. The composition may, for example, be contacted with the teeth for a time period of one second to 20 hours. More preferably from 1 s to 10 hours, more preferably still from 10 s to 1 hour and most preferably from 30 s minute to 5 minutes. The composition may be used daily, for example for use by an individual once, twice or three times per day.

The following examples are provided to facilitate an understanding of the present invention. The examples are not provided to limit the scope of the claims.

### Examples

### Example 1

This example demonstrates the improved deposition on tooth surface of *in situ* formed calcium silicate hydrate (CSH) compared to pre-formed CSH. All ingredients are expressed by weight percent of the total formulation, and as level of active ingredient.

**TABLE 1**

| Ingredient | Samples | | | | |
|---|---|---|---|---|---|
| | **1** | **2** | **3** | **4** | **5** |
| Pre-formed CSH | 9.1 | -- | -- | -- | -- |
| Na₂SiO₃ • 9H₂O | -- | 34.6 | 33.8 | 31.5 | 33.2 |
| Ca(OH)₂ | -- | 4.5 | -- | -- | -- |
| CaSO₄ • 0.5H₂O | -- | -- | -- | -- | 8.5 |
| CaCl₂ | -- | -- | 6.6 | -- | -- |
| Ca(NO₃)₂ • 4H₂O | -- | -- | -- | 13.1 | -- |
| Water | Balance | Balance | Balance | Balance | Balance |

### Preparation of calcium silicate hydrate powder

The pre-formed CSH was prepared using a mixture of calcium hydroxide and sodium silicate in deionised water. The mixture was formed with an initial Ca:Si ratio of 1:2. The mixture was continuously stirred at room temperature (25°C). The pH of the reaction mixture was adjusted to and maintained around 11 using hydrochloric acid. After stirring for 5 hours the reaction mixture was filtered and the cake washed three times with water before again being filtered. The filter cake was dried in an oven at 80°C for 12 hours to yield the final CSH powder.

### Methods

For Samples 2-5, the calcium source and sodium silicate were added in a molar ratio of 1:2 which is the same Ca/Si molar ratio as for the pre-formed CSH.

To evaluate deposition on enamel surface, for Sample 1, a slurry was prepared by mixing 1g of pre-formed CSH with 10mL water and poured onto the enamel surface. For Samples 2-5, the calcium source and silicate source was pre-mixed using vortex and then added onto the enamel surface, water was added at the beginning of brushing.

The bovine enamel blocks were treated with different slurries via brushing following the same protocol. The enamel blocks were brushed with the slurry under a tooth brushing machine equipped with toothbrushes. The load of the tooth brushing was 170 g +/-5 g and the automatic brushing operated at a speed of 150 rpm. After brushing for 3 minutes, the enamel blocks were rinsed with distilled water twice and soaked in simulated oral fluid (SOF) under the condition of a shaking water bath at 37°C and 60.0 rpm. After soaking for about 5 hours, the enamel blocks were brushed with the slurry by machine using the same procedure as in the first step. These steps are considered as a whole treatment cycle. The enamel blocks were treated for 1 and 3 times.

Simulated oral fluid was made by combining the ingredients in Table 2:

**TABLE 2**

| Ingredient | Amount/g |
|---|---|
| NaCl | 16.07 |
| NaHCO₃ | 0.7 |
| KCl | 0.448 |
| K₂HPO₄*3H₂O | 3.27 |
| MgCl₂*6H₂O | 0.622 |
| 1M HCl | 40ml |
| CaCl₂ | 0.1998 |
| Na₂SO₄ | 0.1434 |
| Buffer | Adjust pH to 7.0 |
| Water | Balance to 2L |

### Results

After one treatment, Scanning Electron Microscopy (SEM) images of the enamel block surfaces were taken. From the SEM images, it was apparent that Samples 2-5 gave more uniform and denser deposition on tooth surface than Sample 1 comprising pre-formed CSH.

The cross-section SEM images after three treatments showed that a new layer was formed on the enamel blocks after treated with Sample 2, while Sample 1 treated enamel blocks did not have a new layer formed on their surfaces. Analysis using EDX (Energy Dispersive X-ray Spectroscopy) indentified the elements of Si, Ca and P within the new layer, indicating that calcium silicate (CS) deposited on tooth surface and induced remineralization.

From the electron diffraction pattern obtained by Transmission Electron Microscopy (TEM), it clearly showed that for Sample 2, the new layer formed on the enamel blocks was hydroxyapatite (HAP), which indicated the full transformation of deposited CS into HAP. But in Sample 3, only partial deposited CS was transformed into HAP. The results unexpectedly reveal that insoluble and/or slightly soluble calcium source behaves better than soluble calcium source for tooth remineralization.

### Example 2

This example demonstrates the improved deposition of particles by using *in situ* formed CSH. All ingredients are expressed by weight percent of the total formulation, and as level of active ingredient.

**TABLE 3**

| Ingredient | Samples | | | |
|---|---|---|---|---|
| | **6** | **7** | **8** | **9** |
| Glycerine | Balance | Balance | Balance | Balance |
| Pre-formed CSH | - | 8.3 | - | - |
| Na₂SiO₃•9H₂O | - | - | 32.6 | 29.8 |
| Ca(OH)₂ | - | - | 4.2 | - |
| Ca(NO₃)₂•4H₂O | - | - | - | 12.4 |
| Calcium silicate coated titanium dioxide^{a} | 9.1 | 8.3 | 5.7 | 5.3 |

| | | | | |
|---|---|---|---|---|
| a. Commercially available calcium silicate coated titanium dioxide from KOBO Products Inc | | | | |

### Methods

Fresh slurries were prepared by mixing test samples with water at a ratio of 5 g to 10 mL water, and used immediately.

The bovine enamel blocks were treated with different slurries via brushing following the same protocol. The enamel blocks were brushed with the slurry under a tooth brushing machine equipped with toothbrushes. The load of the tooth brushing was 170 g +/-5 g and the automatic brushing operated at a speed of 150 rpm. After brushing for 3 minutes, the enamel blocks were rinsed with distilled water twice and soaked in SOF for more than 5 hours under the condition of a shaking water bath at 37°C and 60.0 rpm. After soaking for about 5 hours, the enamel blocks were brushed with the slurry by machine using the same procedure as in the first step. These steps are considered as a whole treatment cycle. The enamel blocks were treated for 5 times. The enamel blocks were further brushed with water before taking the cross-section SEM images.

### Results

After five treatments, SEM images of the enamel block surfaces were taken. From the SEM images, it showed that composite particles were randomly attached onto enamel surface for Samples 6 and 7. For Samples 8 and 9, it showed the formation of a new layer and particles or particles aggregates were uniformly embedded in the newly formed layer.

For Samples 6 and 7, after the final water brushing, most attached particles were easily brushed away. The cross-section SEM images were taken after the final water brushing, for sample 8, it clearly showed that the particles remained in the newly formed layer, which indicated the *in situ* formed CSH helps the deposition of particles onto enamel surfaces. Analysis using EDX further indentified the elements of Ti, Ca and P within the new layer, indicating the deposition of particles on tooth surface.

### Example 3

This example demonstrates the amounts of high refractive particles in the compositions for whitening benefits. All ingredients are expressed by weight percent of the total formulation, and as level of active ingredient.

**TABLE 4**

| Ingredient | Samples | | | | | | | |
|---|---|---|---|---|---|---|---|---|
| | **10** | **11** | **12** | **13** | **14** | **15** | **16** | **17** |
| Glycerine | Balance | Balance | Balance | Balance | Balance | Balance | Balance | Balance |
| Na₂SiO₃ | 10 | 10 | 10 | 10 | 10 | 10 | 10 | 10 |
| Ca(OH)₂ | 3 | 3 | 3 | - | - | - | - | - |
| CaCl₂ | - | - | - | 5 | 5 | 5 | 5 | 5 |
| Unmodified titanium dioxide^{b} | 5 | - | - | 2 | 5 | 8 | - | - |
| Calcium silicate coated titanium dioxide^{a} | - | 1 | 2 | - | - | - | 5 | 8 |

| | | | | | | | | |
|---|---|---|---|---|---|---|---|---|
| b. Commercially available unmodified titanium dioxide from KOBO Products Inc | | | | | | | | |

### Methods

Fresh serum slurry were prepared by mixing 5 g test sample with 5 mL water and used immediately.

The bovine enamel blocks were soaked in the slurry for 30 minutes at 37°C. Then the samples were rinsed by water using a tooth brushing machine equipped with toothbrushes on the moving part of a tooth brushing machine (exclude the variance of manual rinse) at a speed of 150 rpm for 1 minute. Then the enamel blocks were incubated in SOF overnight (∼16 hours) in a shaking water bath at 37°C to mimic oral environment. The enamel blocks were brushed with water for 1 minute using a tooth brushing machine equipped with toothbrushes to mimic the abrasion on tooth surface during consumer daily behaviour. The load of the tooth brushing was 170 g +/-5 g and the automatic brushing operated at a speed of 150 rpm. These steps are considered as a whole treatment cycle. The enamel blocks were treated for one, three and five times.

### Results

The results of whitening effect after treatment are summarized in Table 5 (error represents 95% confidence interval for duplicate measurements).

**TABLE 5**

| Change in WIO | Sample 10 | Sample 11 | Sample 12 | Sample 13 | Sample 14 | Sample 15 | Sample 16 | Sample 17 |
|---|---|---|---|---|---|---|---|---|
| One treatment | 0.77±0.536 | -0.58±0.608 | -0.05±0.764 | -1.08±0.640 | -0.66±0.392 | 0.81±0.706 | 0.10±0.908 | 2.55±1.346 |
| Three treatments | 2.85±1.450 | -0.72+0.673 | 0.21±1.483 | -1.13±0.575 | -1.05±0.346 | 0.20±1.477 | -0.61±0.699 | 3.34±1.777 |
| Five treatments | 4.59±1.800 | -0.11±0.510 | -0.14±0.987 | NM^{c} | NM^{c} | 0.30±1.352 | NM^{c} | 3.58±1.673 |

| | | | | | | | | |
|---|---|---|---|---|---|---|---|---|
| c. NM means that the data is not measured. | | | | | | | | |

It can be seen that when calcium hydroxide was used as the calcium source, Sample 10 showed obvious progressive whitening effect while Samples 11 and 12 with low dosage of high refractive index particles showed little whitening effect. When calcium chloride was used as the calcium source, Sample 17 comprising 4% of high refractive index composite particles showed progressive whitening effect while Samples 13 to 16 with unmodified particles or low dosage of composite particles showed little whitening effect.

### Example 4

This example shows an anhydrous oral care composition consistent with this invention.

**TABLE 6**

| Ingredients | Percent by weight |
|---|---|
| Glycerin | 61.44 |
| Sodium monofluorophosphate | 1.11 |
| Sodium saccharin | 0.25 |
| Sodium silicate | 10.00 |
| Calcium hydroxide | 3.00 |
| Calcium silicate coated titanium dioxide^{a} | 15.00 |
| Silica abrasive | 6.00 |
| Sodium lauryl sulfate | 2.00 |
| Flavor | 1.20 |

## Claims

1. An oral care composition comprising:
a) a calcium source;
b) a silicate source that dissolves in water to give a solution with a concentration of at least 0.1 moles per litre at 25°C and atmospheric pressure;
c) from 10% to 30% by weight of high refractive index particles; and
d) a physiologically acceptable carrier;
wherein the particles have a refractive index ranging from 1.9 to 4.0;
wherein the calcium source is calcium hydroxide, calcium gluconate, calcium oxide, calcium lactate, calcium sulfate, calcium carboxymethyl cellulose, calcium alginate, calcium salts of citric acid, calcium chloride, calcium nitrate, calcium acetate or mixtures thereof; and
wherein the silicate source comprises sodium silicate, potassium silicate, tetraethyl orthosilicate, tetraethylsilicate or mixtures thereof.

2. The oral care composition according to claim 1, wherein the calcium source is calcium hydroxide, calcium nitrate, calcium chloride, calcium sulfate or mixtures thereof.

3. The oral care composition according to claim 1 or claim 2, wherein the silicate source is sodium silicate.

4. The oral care composition according to any of the preceding claims, wherein the calcium source is present in an amount ranging from 1 to 20% by total weight of the oral care composition.

5. The oral care composition according to any of the preceding claims, wherein the molar ratio of calcium source to silicate source is from 1:1.5 to 1:4, preferably 1:1.7 to 1:3.

6. The oral care composition according to any of the preceding claims, wherein the high refractive index particles have a refractive index ranging from 2.5 to 4.0.

7. The oral care composition according to claim 6, wherein the high refractive index particles are zinc oxide, titanium dioxide, zirconium dioxide or a mixture thereof, preferably titanium dioxide.

8. The oral care composition according to claim 6, wherein the high refractive index particles are composite particles comprising a first component core comprising a metal compound, preferably the metal is selected from zinc, titanium, zirconium or a mixture thereof, and a second component coating comprising the element calcium, and optionally, potassium, sodium, magnesium, aluminium or a mixture thereof.

9. The oral care composition according to claim 8, wherein the high refractive index particle is titanium dioxide coated with calcium silicate.

10. The oral care composition according to any of the preceding claims, wherein the water content of the composition is less than 1.5%, preferably from 0.0 to 0.75% by total weight of the oral care composition.

11. The oral care composition according to any of the preceding claims, wherein the oral care composition is a monophase anhydrous composition.

12. The oral care composition according to claims 1 to 10, wherein the oral care composition is a dual phase composition, wherein the calcium source and the soluble silicate source are spatially separated prior to use of the composition.

13. An oral care composition according to any of the preceding claims for use in a method of whitening and/or remineralizing the teeth of an individual comprising the step of applying the composition to at least one surface of the teeth of the individual.

14. The oral care composition for use according to claim 13, wherein the calcium source and the soluble silicate source are applied from spatially separated compositions simultaneously or sequentially, preferably simultaneously.

## Patentansprüche

1. Mundpflegezusammensetzung, umfassend:
a) eine Calciumquelle;
b) eine Silikatquelle, die sich in Wasser auflöst, um bei 25°C und Atmosphärendruck eine Lösung einer Konzentration von mindestens 0,1 mol pro Liter zu liefern;
c) von 10 Gewichts-% bis 30 Gewichts-% Partikel mit hohem Brechungsindex; und
d) einen physiologisch verträglichen Träger;
wobei die Partikel einen von 1,9 bis 4,0 reichenden Brechungsindex aufweisen,
wobei die Calciumquelle Calciumhydroxid, Calciumgluconat, Calciumoxid, Calciumlactat, Calciumsulfat, Calciumcarboxymethylcellulose, Calciumalginat, Calciumsalze von Citronensäure, Calciumchlorid, Calciumnitrat, Calciumacetat oder Mischungen davon ist; und
wobei die Silikatquelle Natriumsilikat, Kaliumsilikat, Tetraethylorthosilikat, Tetraethylsilikat oder Mischungen davon umfasst.

2. Mundpflegezusammensetzung nach Anspruch 1, wobei die Calciumquelle Calciumhydroxid, Calciumnitrat, Calciumchlorid, Calciumsulfat oder Mischungen davon ist.

3. Mundpflegezusammensetzung nach Anspruch 1 oder Anspruch 2, wobei die Silikatquelle Natriumsilikat ist.

4. Mundpflegezusammensetzung nach irgendeinem der vorhergehenden Ansprüche, wobei die Calciumquelle in einer Menge vorliegt, die von 1 bis 20 Gewichts-%, bezogen auf das Gesamtgewicht der Mundpflegezusammensetzung, reicht.

5. Mundpflegezusammensetzung nach irgendeinem der vorhergehenden Ansprüche, wobei das Molverhältnis der Calciumquelle zur Silikatquelle von 1:1,5 bis 1:4, vorzugsweise von 1:1,7 bis 1:3, beträgt.

6. Mundpflegezusammensetzung nach irgendeinem der vorhergehenden Ansprüche, wobei die Partikel mit hohem Brechungsindex einen Brechungsindex haben, der von 2,5 bis 4,0 reicht.

7. Mundpflegezusammensetzung nach Anspruch 6, wobei die Partikel mit hohem Brechungsindex Zinkoxid, Titandioxid, Zirconiumdioxid oder eine Mischung davon, vorzugsweise Titandioxid, sind.

8. Mundpflegezusammensetzung nach Anspruch 6, wobei die Partikel mit hohem Brechungsindex Verbundpartikel sind, umfassend einen Kern als ersten Bestandteil, der eine Metallverbindung umfasst, wobei das Metall vorzugsweise unter Zink, Titan, Zirconium oder einer Mischung davon ausgewählt ist, und eine Beschichtung als zweiten Bestandteil, die das Element Calcium und gegebenenfalls Kalium, Natrium, Magnesium, Aluminium oder eine Mischung davon umfasst.

9. Mundpflegezusammensetzung nach Anspruch 8, wobei das Partikel mit hohem Brechungsindex Titandioxid, beschichtet mit Calciumsilikat, ist.

10. Mundpflegezusammensetzung nach irgendeinem der vorhergehenden Ansprüche, wobei der Wassergehalt der Zusammensetzung weniger als 1,5%, vorzugsweise von 0,0 bis 0,75%, bezogen auf das Gesamtgewicht der Mundpflegezusammensetzung, beträgt.

11. Mundpflegezusammensetzung nach irgendeinem der vorhergehenden Ansprüche, wobei die Mundpflegezusammensetzung eine einphasige wasserfreie Zusammensetzung ist.

12. Mundpflegezusammensetzung nach den Ansprüchen 1 bis 10, wobei die Mundpflegezusammensetzung eine zweiphasige Zusammensetzung ist, wobei die Calciumquelle und die lösliche Silikatquelle vor der Benutzung der Zusammensetzung räumlich getrennt vorliegen.

13. Mundpflegezusammensetzung nach irgendeinem der vorhergehenden Ansprüche zur Verwendung in einem Verfahren des Aufhellens und/oder Remineralisierens von Zähnen eines Individuums, umfassend den Schritt des Auftragens der Zusammensetzung auf mindestens eine Oberfläche der Zähne des Individuums.

14. Mundpflegezusammensetzung zur Verwendung nach Anspruch 13, wobei die Calciumquelle und die lösliche Silicatquelle aus räumlich getrennten Zusammensetzungen gleichzeitig oder nacheinander, vorzugsweise gleichzeitig, angewendet werden.

## Revendications

1. Composition pour le soin oral comprenant :
a) une source de calcium ;
b) une source de silicate qui se dissout dans l'eau pour fournir une solution avec une concentration d'au moins 0,1 mole par litre à 25°C et pression atmosphérique ;
c) de 10 % à 30 % en masse de particules d'indice de réfraction élevée ; et
d) un support physiologiquement acceptable ;
dans laquelle les particules présentent un indice de réfraction de 1,9 à 4,0 ;
dans laquelle la source de calcium est l'hydroxyde de calcium, gluconate de calcium, oxyde de calcium, lactate de calcium, sulfate de calcium, calcium carboxyméthylcellulose, alginate de calcium, sels de calcium d'acide citrique, chlorure de calcium, nitrate de calcium, acétate de calcium ou mélanges de ceux-ci ; et
dans laquelle la source de silicate comprend du silicate de sodium, silicate de potassium, orthosilicate de tétraéthyle, silicate de tétraéthyle ou mélanges de ceux-ci.

2. Composition pour le soin oral selon la revendication 1, dans laquelle la source de calcium est l'hydroxyde de calcium, nitrate de calcium, chlorure de calcium, sulfate de calcium ou mélanges de ceux-ci.

3. Composition pour le soin oral selon la revendication 1 ou la revendication 2, dans laquelle la source de silicate est le silicate de sodium.

4. Composition pour le soin oral selon l'une quelconque des revendications précédentes, dans laquelle la source de calcium est présente dans une quantité de 1 à 20 % en masse totale de la composition pour le soin oral.

5. Composition pour le soin oral selon l'une quelconque des revendications précédentes, dans laquelle le rapport molaire de source de calcium à source de silicate est de 1:1,5 à 1:4, de préférence de 1:1,7 à 1:3.

6. Composition pour le soin oral selon l'une quelconque des revendications précédentes, dans laquelle les particules d'indice de réfraction élevé présentent un indice de réfraction de 2,5 à 4,0.

7. Composition pour le soin oral selon la revendication 6, dans laquelle les particules d'indice de réfraction élevé sont l'oxyde de zinc, dioxyde de titane, dioxyde de zirconium ou un mélange de ceux-ci, de préférence le dioxyde de titane.

8. Composition pour le soin oral selon la revendication 6, dans laquelle les particules d'indice de réfraction élevée sont des particules composites comprenant un noyau de premier constituant comprenant un composé de métal, le métal est de préférence choisi parmi le zinc, titane, zirconium ou un mélange de ceux-ci, et un revêtement de second constituant comprenant l'élément calcium, et éventuellement, potassium, sodium, magnésium, aluminium ou un mélange de ceux-ci.

9. Composition pour le soin oral selon la revendication 8, dans laquelle la particule d'indice de réfraction élevé est du dioxyde de titane revêtu avec du silicate de calcium.

10. Composition pour le soin oral selon l'une quelconque des revendications précédentes, dans laquelle la teneur en eau de la composition est inférieure à 1,5 %, de préférence de 0,0 à 0,75 % en masse totale de la composition pour le soin oral.

11. Composition pour le soin oral selon l'une quelconque des revendications précédentes, dans laquelle la composition pour le soin oral est une composition anhydre monophasique.

12. Composition pour le soin oral selon l'une quelconque des revendications 1 à 10, dans laquelle la composition pour le soin oral est une composition à phase double, dans laquelle la source de calcium et la source de silicate soluble sont spatialement séparées avant l'utilisation de la composition.

13. Composition pour le soin oral selon l'une quelconque des revendications précédentes pour une utilisation dans un procédé de blanchiment et/ou de reminéralisation des dents d'un individu comprenant l'étape d'application de la composition à au moins une surface des dents de l'individu.

14. Composition pour le soin oral selon la revendication 13, dans laquelle la source de calcium et la source de silicate soluble sont appliquées à partir de compositions spatialement séparées simultanément ou successivement, de préférence simultanément.
